# EUROPÄISCHE PATENTSCHRIFT

(11) **EP 2 704 655 B1**
(45) Veröffentlichungstag und Bekanntmachung des Hinweises auf die Patenterteilung: **17.07.2024**
(21) Anmeldenummer: 12726016.4
(22) Anmeldetag: 04.05.2012
(51) Int. Cl.: A61B 18/04, A61L 2/14, A61B 18/00, H05H 1/46, A61N 5/06

(54) **VERFAHREN ZUR INAKTIVIERUNG VON MOLEKÜLEN UND VORRICHTUNG ZU DESSEN DURCHFÜHRUNG**
METHOD FOR DEACTIVATING MOLECULES AND DEVICE FOR CARRYING OUT SAID METHOD
PROCÉDÉ D'INACTIVATION DE MOLÉCULES ET DISPOSITIF PERMETTANT LA MISE EN OEUVRE DUDIT PROCÉDÉ

(30) Priorität: 05.05.2011 DE 102011100751; 19.11.2011 DE 102011118996
(43) Veröffentlichungstag der Anmeldung: 12.03.2014
(60) Teilanmeldung: 23208872.4
(73) Patentinhaber: Max-Planck-Gesellschaft zur Förderung der Wissenschaften e.V., 80539 München (DE)
(72) Erfinder: MORFILL, Gregor, 81925 München (DE); SHIMIZU, Tetsuji, 85748 Garching (DE); ZIMMERMANN, Julia, 81925 München (DE); LI, Yangfang, 82140 Olching (DE); STEFFES, Bernd, 82487 Oberammergau (DE)
(74) Vertreter: Kordel, Mattias
(86) Internationale Anmeldenummer: PCT/EP2012/001923
(87) Internationale Veröffentlichungsnummer: WO 2012/150040

(56) Entgegenhaltungen:
- WO-A2-2009/021919
- WO-A2-2009/067682
- DE-B3- 10 254 135
- DE-U1- 202009 011 521
- GB-A- 2 454 461
- US-A1- 2007 253 865
- US-A1- 2008 193 329
- US-A1- 2011 022 043
- US-A1- 2011 034 914
- GREGORY FRIDMAN ET AL: "Applied plasma medicine", PLASMA PROCESSES AND POLYMERS, WILEY - VCH VERLAG, DE, vol. 5, no. 6, 15 August 2008 (2008-08-15), pages 503 - 533, XP002617381, ISSN: 1612-8850, [retrieved on 20080416], DOI: 10.1002/PPAP.200700154

## Beschreibung

Die Erfindung betrifft ein Verfahren zur Inaktivierung von Molekülen gemäß Anspruch 1 und eine Vorrichtung zur Durchführung eines Verfahrens zur Inaktivierung von Molekülen gemäß Anspruch 5.

Es ist bekannt, dass ein Plasma zur Desinfektion insbesondere mit Bakterien kontaminierter Oberflächen verwendet werden kann. Es sind Verfahren und Vorrichtungen bekannt, mithilfe derer Oberflächen durch Plasma dekontaminiert beziehungsweise desinfiziert werden. Dabei werden Bakterien, Keime, Viren, Sporen, Pilze und andere Elemente, welche die Oberfläche unsteril machen, durch das Plasma abgetötet beziehungsweise inaktiviert. Die bekannten Verfahren und Vorrichtungen sind jedoch nicht auf die Anwendung auf Oberflächen beschränkt. Auch beispielsweise Luftvolumina können in der genannten Weise desinfiziert werden.

Dokument US2008193329 offenbart ein Verfahren und ein System zur nicht-thermischen Plasmabehandlung von Material bei hohem Druck. Die Plasmabehandlung kann z.B. zur Oberflächenbehandlung von Werkstoffen und zur Gasreinigung eingesetzt werden.

Für die Bildung unangenehmer Gerüche auf Oberflächen oder beispielsweise auch in einem Luftvolumen sind vielfach Bakterien verantwortlich, welche vorhandene Nährstoffe metabolisieren und dabei unangenehm riechende Substanzen erzeugen. Durch die Abtötung oder Inaktivierung dieser Bakterien kann zumindest vorübergehend verhindert werden, dass weitere unangenehm riechende Substanzen oder auch andere störende Moleküle gebildet werden. Die bereits vorhandenen Substanzen werden so allerdings nicht entfernt, sodass ihr Geruch üblicherweise durch Zusatzstoffe maskiert beziehungsweise überdeckt werden muss. Es ist wünschenswert, diese Substanzen zu inaktivieren. Es ist auch wünschenswert, andere störende, insbesondere oberflächenassoziierte Moleküle zu inaktivieren.

Der Erfindung liegt daher die Aufgabe zugrunde, ein Verfahren und eine Vorrichtung anzugeben, mit deren Hilfe insbesondere geruchsrelevante Moleküle inaktiviert werden können.

Diese Aufgabe wird gelöst, indem das Verfahren zur Inaktivierung von Molekülen gemäß Anspruch 1 angegeben wird. Dabei spricht der Begriff "Inaktivierung" an, dass die Moleküle beispielsweise durch die heißen Elektronen dissoziiert oder in ihrer molekularen Struktur verändert werden, oder dass durch die heißen Elektronen Reaktionen, beispielsweise auch Anlagerungsreaktionen, ermöglicht werden, durch welche die Moleküle so verändert werden, dass sie jedenfalls nicht mehr geruchsrelevant oder anderweitig störend sind. Der Begriff "geruchsrelevant" spricht an, dass die Moleküle selbst unangenehm riechen, mit weiteren Molekülen so reagieren können, dass unangenehme Gerüche entstehen, sodass sie also geruchsbildend sind, und/oder dass sie als Nährstoff für Bakterien dienen, die hieraus wiederum geruchsrelevante Moleküle synthetisieren. Der Begriff "Moleküle" umfasst auch geruchsrelevante oder anderweitig störende Substanzen, welche nicht molekular vorliegen. Der Begriff "heiße Elektronen" spricht Elektronen an, die im unmittelbaren Bereich des Plasmas, also des quasi-neutralen, ionisierten Gases beziehungsweise Aggregatzustands vorliegen, und die eine Geschwindigkeitsverteilung aufweisen, die entweder nichtthermisch ist, wobei eine mittlere Geschwindigkeit der Elektronen einer kinetischen Energie von mindestens einem, vorzugsweise mehreren Elektronenvolt entspricht, oder deren Geschwindigkeitsverteilung thermisch ist, wobei die der Verteilung zuordenbare Temperatur deutlich über 25°C und vorzugsweise so hoch liegt, dass die mittlere kinetische Energie der Elektronen ein Elektronenvolt bis einige Elektronenvolt beträgt. Jedenfalls reicht die mittlere kinetische Energie der Elektronen vorzugsweise aus, um insbesondere geruchsrelevante Moleküle zu inaktivieren. Besonders bevorzugt reicht die mittlere kinetische Energie der Elektronen aus, um insbesondere unangenehm riechende Moleküle zu dissoziieren. Es zeigt sich, dass gerade große Moleküle Dissoziationsquerschnitte beziehungsweise Dissoziationsenergien aufweisen, die für das Verfahren geeignet sind. Es ist also insbesondere Möglich, große Moleküle, wie beispielsweise Proteinmoleküle, zu inaktivieren.

Bevorzugt werden auf einer Oberfläche angelagerte, vorzugsweise geruchsrelevante Moleküle inaktiviert, insbesondere bevor diese sich in der Umgebung verteilen können. Häufig sind Oberflächen, beispielsweise Hautoberflächen, mit geruchsrelevanten oder anderweitig störenden Molekülen belastet, die quasi oberflächenassoziiert sind. Diese werden besonders vorteilhaft mithilfe des Verfahrens inaktiviert. Das Verfahren ist in diesem Fall besonders wirksam, weil mithilfe eines gegebenen Elektronenflusses eine gegebene Oberflächendichte von Molekülen aufgrund der relevanten Wirkungsquerschnitte einfacher und in kürzerer Zeit reduziert werden kann, als dies bei einer entsprechenden Volumendichte der Moleküle der Fall ist.

Besonders bevorzugt wird das Plasma in einem Abstand zu der zu behandelnden Oberfläche erzeugt, wobei der Abstand so gewählt ist, dass eine Einwirkung der heißen Elektronen auf die oberflächenassoziierten Moleküle gewährleistet ist. Dies spricht an, dass die Dichte beziehungsweise der Fluss heißer Elektronen mit dem Abstand zu einer Plasmaquelle beziehungsweise zum Entstehungsort des eigentlichen Plasmas stark abnimmt. Der Erzeugungsort des Plasmas muss daher so nahe an die zu behandelnde Oberfläche heranverlagert werden, dass genügend heiße Elektronen für die Inaktivierung der oberflächenassoziierten Moleküle zur Verfügung stehen.

Besonders bevorzugt wird ein Verfahren, bei dem zusätzlich zu der Inaktivierung vorzugsweise geruchsrelevanter Moleküle auch Bakterien, vorzugsweise geruchsbildende Bakterien oder Bakterien, die andere störende Moleküle bilden, durch das Plasma abgetötet werden. Der Begriff "Plasma" schließt hierbei nicht nur das Plasma im engeren Sinne ein, also dass quasi-neutrale, zumindest teilweise ionisierte Gas, welches die heißen Elektronen umfasst, sondern auch durch das Plasma erzeugte reaktive Spezies, beispielsweise angeregte und/oder reaktive Moleküle, Radikale und weitere aktive Spezies, deren Reichweite größer ist als die der heißen Elektronen. Insbesondere solche reaktiven Spezies, die dem Plasma im weiteren Sinne zuzurechnen sind, können Bakterien insbesondere auf einer zu behandelnden Oberfläche abtöten. Dadurch wird neben der Inaktivierung vorzugsweise geruchsrelevanter Moleküle zugleich vermieden, dass neue unerwünschte Moleküle durch den Metabolismus gegebenenfalls vorhandener Bakterien gebildet werden.

In diesem Zusammenhang wird auch ein nicht zur Erfindung gehörendes Verfahren beschrieben, bei dem der Abstand der Plasmaerzeugung zu einer zu behandelnden Oberfläche abhängig von einem gewünschten Wirkmechanismus gewählt wird. Insbesondere kann durch die Wahl des Abstandes ein dominanter Wirkmechanismus eingestellt werden: Wird der Abstand kleiner gewählt, dominiert der Inaktivierungseffekt insbesondere geruchsrelevanter Moleküle durch die heißen Elektronen. Wird der Abstand dagegen größer gewählt, dominiert der bakterizide Effekt aufgrund der von dem Plasma im weiteren Sinne umfassten reaktiven Spezies. Bevorzugt wird der Abstand so gewählt, dass beide Effekte möglichst wirksam sind.

Bei dem erfindungsgemäßen Verfahren ist die Oberfläche ausgewählt aus textilem Material, Keramik, Kunststoff oder Leder Zugleich kann optional ein auf die zu behandelnde Oberfläche aufbringbarer Zusatzstoff ausgewählt sein aus mindestens einem Deodorant und/oder mindestens einem Antitranspirant. Dabei spricht der Begriff "Deodorant" Substanzen an, die im Wesentlichen der Maskierung, Überdeckung oder Inaktivierung geruchsrelevanter Substanzen dient. Der Begriff "Antitranspirant" spricht Substanzen an, die im Wesentlichen dazu dienen, eine Schweißbildung zu verhindern, beispielsweise indem Schweißporen umfassende Hautbereiche adstringiert werden.

Auch können zusätzlich nicht-geruchsrelevante Moleküle inaktiviert werden, insbesondere solche, die Unbehagen, Unwohlsein, Krankheit, Schwächung oder ähnliche Zustände verursachen. Dies sind in einer Ausführungsform Allergene, Proteinmoleküle oder Prionen. Solche Moleküle werden hier auch kurz als "störende Moleküle" bezeichnet. Der Begriff "nicht-geruchsrelevant" bedeutet, dass der störende Effekt dieser Moleküle zumindest nicht in erster Linie darin besteht, dass sie unangenehm riechen. Dabei ist jedoch keinesfalls ausgeschlossen, dass die hier angesprochenen Moleküle neben ihrer primären Wirkung zusätzlich einen unangenehmen Geruch aufweisen.

Schließlich wird ein nicht zur Erfindung gehörendes Verfahren beschrieben, bei welchem Moleküle in einem Fluidstrom einer Vorrichtung inaktiviert werden, die zur Erzeugung und/oder Durchleitung eines Fluidstroms vorgesehen ist. Die Vorrichtung ist dabei vorzugsweise als Abzugshaube, insbesondere Dunstabzugshaube, Gasreinigungseinrichtung, Vorrichtung zur Durchleitung von Flüssigkeiten, insbesondere Zapfanlage oder Wasserhahn, Staubsauger, Föhn, Dampfstrahler oder Handtrockner ausgebildet. Es ist damit möglich, störende Moleküle, seien sie geruchsrelevant oder in anderer Weise unangenehm oder gar gesundheitsgefährdend, unmittelbar in dem Fluidstrom einer solchen Vorrichtung zu inaktivieren, bevor sie ihrer eigentlichen Bestimmung beziehungsweise ihrem Bestimmungsort zugeführt werden.

Weitere bevorzugte Ausgestaltungen ergeben sich aus den Unteransprüchen.

Die Aufgabe wird auch gelöst, indem eine Vorrichtung mit den Merkmalen des Anspruchs 5 geschaffen wird. Diese dient der Durchführung eines Verfahrens zur Inaktivierung von Molekülen und umfasst ein Gehäuse, eine dem Gehäuse zugeordnete Plasmaquelle, und mindestens ein Abstandshaltemittel. Das mindestens eine Abstandshaltemittel ist so an dem Gehäuse und/oder der Plasmaquelle vorgesehen, dass zumindest bereichsweise ein Abstand zwischen der Plasmaquelle und einer zu behandelnden Oberfläche einhaltbar ist, bei welchem an der zu behandelnden Oberfläche angelagerte Moleküle durch heiße Elektronen des Plasmas inaktiviert werden. Bezüglich der Begriffsbestimmung wird auf die Ausführungen zu dem Verfahren verwiesen. Wesentlich ist, dass das Abstandshaltemittel so vorgesehen ist, dass zumindest bereichsweise ein bereits in Zusammenhang mit dem Verfahren beschriebener Abstand eingehalten werden kann, bei welchem ein ausreichender Fluss an heißen Elektronen zur Verfügung steht, um oberflächenassoziierte, vorzugsweise geruchsrelevante Moleküle zu inaktivieren.

Bevorzugt ist das Abstandshaltemittel einstellbar ausgebildet. Damit ist es möglich, einen Wirkmechanismus oder zumindest einen dominanten Wirkmechanismus auszuwählen. Dabei kann der Schwerpunkt der Behandlung mithilfe der Vorrichtung entweder auf die Inaktivierung vorzugsweise geruchsrelevanter Moleküle oder auf den im Zusammenhang mit dem Verfahren beschriebenen bakteriziden Effekt gelegt werden.

Bevorzugt weist die Plasmaquelle eine Oberfläche auf, in deren Bereich das Plasma erzeugt wird.

Diese Oberfläche kann bevorzugt gekrümmt, vorzugsweise konvex oder konkav ausgebildet sein. In diesem Fall ergibt sich bereits durch die Krümmung der Oberfläche vorzugsweise zumindest bereichsweise ein Abstand zu der zu behandelnden Oberfläche, der einem in Hinblick auf die genannten Wirkmechanismen gewünschten Abstand entspricht, wenn die Oberfläche der Plasmaquelle auf der zu behandelnden Oberfläche zumindest bereichsweise aufliegt.

Bevorzugt sind die Oberfläche der Plasmaquelle und das Abstandshaltemittel so angeordnet, dass sie zusammen mit einer behandelnden Oberfläche ein geschlossenes Volumen einschließen können. Es ergibt sich so eine besonders effiziente und rasche Behandlung der Oberflächenbereiche, die das geschlossene Volumen abgrenzen.

Besonders bevorzugt ist das geschlossene Volumen einstellbar, indem die elastische Oberfläche der Plasmaquelle flexibel und/oder elastisch ausgebildet ist und mit dem einstellbaren Abstandshalter entsprechend zusammenwirkt. Durch eine Einstellung des Abstandshalters ist dann die flexible beziehungsweise elastische Oberfläche der Plasmaquelle beispielsweise biegbar, dehnbar oder reduzierbar, sodass letztlich das mit der zu behandelnden Oberfläche einschließbare Volumen eingestellt wird.

Besonders bevorzugt ist auch ein Applikationsmittel vorgesehen, das geeignet beziehungsweise ausgebildet ist, um einen vorzugsweise flüssigen Zusatzstoff, beispielsweise ein Deodorant und/oder Antitranspirant auf eine zu behandelnde Oberfläche aufzubringen.

Schließlich wird noch bevorzugt, dass das Applikationsmittel ein Spray, eine Rollervorrichtung oder eine Stick- beziehungsweise Stiftvorrichtung umfasst.

Weitere vorteilhafte Ausgestaltungen ergeben sich aus den Unteransprüchen.

Es wird auch eine nicht zur Erfindung gehörende Vorrichtung mit einer Plasmaquelle beschrieben, wobei die Vorrichtung zur Durchleitung und/oder Erzeugung eines Fluidstroms ausgebildet ist. Die Plasmaquelle ist so ausgebildet und/oder angeordnet, dass Moleküle in dem Fluidstrom inaktiviert werden können.

Diese nicht zur Erfindung gehörende Vorrichtung kann als Abzugshaube, insbesondere Dunstabzugshaube, Gasreinigungseinrichtung, Staubsauger, Föhn, Dampfstrahler, Vorrichtung zur Durchleitung von Flüssigkeiten, insbesondere Zapfanlage oder Wasserhahn, oder Handtrockner ausgebildet sein. Mit Hilfe der Vorrichtung ist es möglich, geruchsrelevante oder in anderer Form unangenehme, gegebenenfalls sogar gesundheitsschädliche Moleküle in dem Fluidstrom zu inaktivieren, bevor dieser seiner eigentlichen Bestimmung zugeführt wird beziehungsweise seinen Bestimmungsort erreicht.

Die Erfindung wird im Folgenden anhand der Zeichnung näher erläutert. Es zeigen:
- Figur 1: eine schematische Ansicht eines ersten Ausführungsbeispiels einer Vorrichtung zur Durchführung eines Verfahrens zur Inaktivierung vorzugsweise geruchsrelevanter Moleküle;
- Figur 2: eine schematische Ansicht eines zweiten Ausführungsbeispiels der Vorrichtung;
- Figur 3: eine schematische Ansicht eines dritten Ausführungsbeispiels der Vorrichtung;
- Figur 4: eine schematische Ansicht eines vierten Ausführungsbeispiels der Vorrichtung, und
- Figur 5: eine schematische Ansicht eines fünften Ausführungsbeispiels der Vorrichtung.

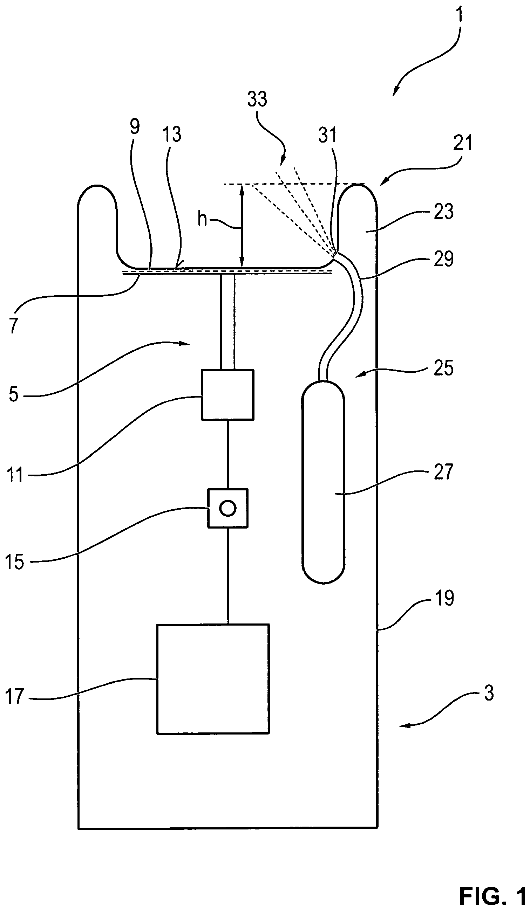

Figur 1 zeigt eine schematische Darstellung eines ersten Ausführungsbeispiels einer Vorrichtung, die zur Durchführung eines Verfahrens zur Inaktivierung vorzugsweise geruchsrelevanter Moleküle geeignet ist. Dabei wird durch die Vorrichtung ein Plasma erzeugt, wobei vorzugsweise geruchsrelevante Moleküle durch Einwirkung heißer Elektronen des Plasmas auf die zu inaktivierenden Moleküle inaktiviert werden.

Die Vorrichtung 1 umfasst ein Gehäuse 3 und eine dem Gehäuse zugeordnete Plasmaquelle 5. Diese ist hier in dem Gehäuse 3 angeordnet. Es ist vorzugsweise auch möglich, die Plasmaquelle 5 an dem Gehäuse 3 anzuordnen, sodass dieses eher als Träger für die Plasmaquelle 5 dient.

Plasmaquellen sind allgemein bekannt. Es ist möglich, eine Plasmaquelle 5 zu verwenden, die lediglich eine mit einer Wechselspannung, vorzugsweise einer hochfrequenten Wechselspannung beaufschlagte Elektrode umfasst. In diesem Fall dient die zu behandelnde Oberfläche als Gegenelektrode, wobei zwischen den Elektroden ein Plasma gezündet wird. Dies entspricht dem Prinzip der dielektrischen Barriereentladung (dielectric barrier discharge - DBD). Erfindungsgemäß wird eine Plasmaquelle verwendet, die zwei Elektroden umfasst, von denen eine mit einer Wechselspannung beaufschlagt wird, wobei die andere geerdet ist. Um die Sicherheit eines Benutzers zu gewährleisten, ist dabei vorzugsweise die geerdete Elektrode an einer der zu behandelnden Oberfläche zugewandten Oberfläche vorgesehen. Die beiden Elektroden sind durch ein Dielektrikum voneinander beabstandet. Zwischen den Elektroden wird eine Entladung gezündet, sodass auf der Seite der Elektrode, die der zu behandelnden Oberfläche zugewandt ist, ein Plasma gebildet wird, welches auf die zu behandelnde Oberfläche einwirkt. Dies entspricht dem Prinzip der Oberflächen-Mikroentladung (surface micro discharge - SMD). Alternativ ist es auch möglich, eine Plasmaquelle vorzusehen, bei der beide Elektroden in mindestens ein Dielektrikum eingebettet sind. Dabei ist die der zu behandelnden Oberfläche zugewandte Elektrode so nah an der Oberfläche des Dielektrikums vorgesehen, dass ein Plasma auf dieser Oberfläche gebildet wird, wenn einer Entladung zwischen den Elektroden gezündet wird. Vorzugsweise ist auch in diesem Fall die Elektrode geerdet, welche der zu behandelnden Oberfläche zugewandt ist. Da beide Elektroden in mindestens ein Dielektrikum eingebettet sind, ist keine Elektrode frei zugänglich. Dies entspricht dem Prinzip der selbststerilisierenden Oberfläche (self sterilizing surface - SSS).

In den Ausführungsbeispielen, die in den Figuren dargestellt sind, ist jeweils eine Plasmaquelle schematisch angedeutet, die dem Prinzip der selbststerilisierenden Oberfläche genügt. Dies entspricht einer bevorzugten Ausführungsform; gleichwohl ist es ohne Weiteres bevorzugt auch möglich, eine andere Plasmaquelle vorzusehen.

Die Plasmaquelle 5 umfasst eine erste Elektrode 7 und eine zweite Elektrode 9. Beide Elektroden sind mit einer Spannungsquelle 11 verbunden, sodass bevorzugt die erste Elektrode 7 mit einer Hochspannung geeigneter Frequenz beaufschlagt wird, wobei die zweite Elektrode 9 bevorzugt geerdet ist. Beide Elektroden 7, 9 sind bevorzugt in ein Dielektrikum eingebettet, wobei die zweite Elektrode 9 etwas unterhalb einer Oberfläche 13 angeordnet ist, in deren Bereich ein Plasma erzeugt wird, wenn eine Entladung zwischen den Elektroden 7, 9 gezündet wird.

Bevorzugt umfasst die Vorrichtung 1 einen Schalter 15, über den die Plasmaquelle 5 aktivierbar beziehungsweise deaktivierbar ist. Sie umfasst weiterhin bevorzugt eine Energiequelle 17, durch welche die Spannungsquelle 11 mit Energie versorgbar ist. Die Energiequelle 17 kann - wie hier beispielhaft dargestellt - als Batterie, Akkumulator oder sonstige dem Gehäuse 3 zugeordnete Energiequelle ausgebildet sein. Bevorzugt ist es aber auch möglich, einen Anschluss für eine externe Energiequelle, beispielsweise einen Stecker vorzusehen. Ebenfalls ist es möglich, die Plasmaquelle 5 über eine Energieerntevorrichtung (energy-harvesting device) mit Energie zu versorgen. Hier kommen beispielsweise Piezokristalle, in Spulen verschiebliche Permanentmagnete, Vorrichtungen, die auf dem Prinzip der Thermoelektrizität, beispielsweise Seebeck-Effekt, Peltier-Effekt und/oder Thomson-Effekt, basieren, Solarpaneele und zahlreiche andere an sich bekannte Vorrichtungen in Betracht. Diese sind teilweise bevorzugt in die Spannungsquelle 11 integriert oder ersetzen diese. Beispielsweise kann ein Piezokristall vorgesehen sein, welcher zugleich als Spannungsquelle und als Energiequelle dient.

Falls die Vorrichtung 1 als tragbares beziehungsweise Handgerät ausgebildet ist, weist sie bevorzugt einen Griffbereich 19 auf. Besonders bevorzugt ist in diesem Bereich eine rutschfeste Oberfläche vorgesehen, sodass ein Benutzer die Vorrichtung 1 sicher greifen kann.

Die Vorrichtung 1 umfasst außerdem ein Abstandshaltemittel 21. Bei dem dargestellten Ausführungsbeispiel ist dieses so an dem Gehäuse 3 vorgesehen, dass ein Abstand zwischen der Plasmaquelle 5 (hier konkret der Oberfläche 13) und einer nicht dargestellten zu behandelnden Oberfläche einhaltbar ist. Bevorzugt stützt sich die Vorrichtung 1 mithilfe des Abstandshaltemittels 21 an der nicht dargestellten zu behandelnden Oberfläche ab, sodass ein entsprechender Abstand eingehalten wird. Dieser Abstand ist hier durch die Höhe h definiert, um die der höchste Punkt des Abstandshaltemittels 21 über die Oberfläche 13 ragt. Der Abstand beziehungsweise die Höhe h ist so gewählt, dass an der zu behandelnden Oberfläche angelagerte Moleküle durch heiße Elektronen des Plasmas inaktiviert werden. Dazu beträgt der Abstand vorzugsweise zwischen 0 bis 4 mm, bevorzugt zwischen 1 bis 3 mm, und besonders bevorzugt 2 mm. Bevorzugt wird auch ein Abstand, der weniger als 2 mm beträgt. Es ist dann sichergestellt, dass der die zu behandelnde Oberfläche erreichende Fluss heißer Elektronen hinreichend groß ist, um dort assoziierte, insbesondere geruchsrelevante oder anderweitig störende Moleküle zu inaktivieren. Der in Figur 1 rein schematisch dargestellte Abstand beziehungsweise die Höhe h sind im Verhältnis zur Gesamtgröße der Vorrichtung 1 tendenziell übertrieben dargestellt.

Das Abstandshaltemittel 21 ist bei dem in Figur 1 dargestellten Ausführungsbeispiel als Kragen 23 ausgebildet. Dieser ist bevorzugt einstückig mit dem Gehäuse 3 ausgebildet. Bevorzugt ist es allerdings auch möglich, das Abstandshaltemittel 21 von dem Gehäuse 3 getrennt auszubilden und in geeigneter Weise an diesem anzuordnen beziehungsweise zu befestigen.

Die in Figur 1 dargestellte Vorrichtung ist vorzugsweise zylindersymmetrisch ausgebildet. In diesem Fall erstreckt sich bevorzugt auch der Kragen 23 vollständig in Umfangsrichtung der Vorrichtung 1.

Bevorzugt ist es auch möglich, dass quasi statt des ringförmigen Kragens 23 einzelne Abstandshalterelemente an dem Gehäuse 3 angeordnet werden, die bevorzugt symmetrisch, das heißt mit konstantem Winkelabstand - entlang der Umfangsrichtung gesehen - verteilt sind. Beispielsweise können zwei gegenüberliegende Abstandshalterelemente vorgesehen sein. Es ist auch möglich, mehr als zwei Abstandshalterelemente vorzusehen.

Der ringförmig geschlossene Kragen 23 ist als Abstandshaltemittel 21 allerdings insoweit vorteilhaft, als in diesem Fall die Oberfläche 13 und das Abstandshaltemittel 21 so angeordnet sind, dass durch sie und eine zu behandelnde Oberfläche ein geschlossenes Volumen einschließbar ist. Dies ist insbesondere dann der Fall, wenn die zu behandelnde Oberfläche auf der höchsten Erhebung des Abstandshaltemittels 21 - in Umfangsrichtung gesehen - vollständig aufliegt. In diesem Fall ist eine besonders rasche und effiziente Behandlung der Oberfläche möglich, weil sich keinerlei Störeinflüsse wie beispielsweise von außen wirkende Luftströmungen ergeben.

Die Vorrichtung 1 umfasst bevorzugt noch ein Applikationsmittel 25 zum Aufbringen eines vorzugsweise flüssigen Zusatzstoffs auf die zu behandelnde Oberfläche. Bei dem dargestellten Ausführungsbeispiel ist das Applikationsmittel 25 als Spray ausgebildet, welches einen Vorratsbehälter 27, eine Zuleitung 29 und einen Auslass 31 umfasst. Nicht dargestellt ist eine Pumpvorrichtung, welche das Applikationsmittel 25 vorzugsweise umfasst, um einen Zusatzstoff aus dem Vorratsbehälter 27 über die Zuleitung 29 durch den Auslass 31 auszutreiben. Der Vorratsbehälter 27 ist bevorzugt nachfüllbar und/oder austauschbar ausgebildet beziehungsweise in oder an dem Gehäuse 3 angeordnet. Mithilfe des Applikationsmittels 25 ist es bevorzugt möglich, vor, nach oder auch während einer Behandlung der zu behandelnden Oberfläche mithilfe der Plasmaquelle 5 mindestens einen Zusatzstoff auf die Oberfläche aufzubringen. Schematisch angedeutet ist hier ein Sprühnebel 33 des aus dem Auslass 31 ausgetriebenen mindestens einen Zusatzstoffs. Es ist möglich, das Applikationsmittel 25 gemeinsam mit der Plasmaquelle 5 über den Schalter 15 zu aktivieren. Bei einem anderen Ausführungsbeispiel ist vorgesehen, das Applikationsmittel 25 getrennt über eine eigene Aktivierungsvorrichtung zu aktivieren. Dabei sind ein kontinierlicher Betrieb oder auch ein Pulsbetrieb, beispielsweise nach Art eines Pumpsprays, für das Applikationsmittel 25 möglich.

Das Applikationsmittel 25 kann auch als Rollervorrichtung nach Art eines Deorollers, oder auch als Stickvorrichtung beziehungsweise Stiftvorrichtung ausgebildet sein, bei der ein Stick oder Stift zur Applikation mindestens eines Zusatzstoffs vorgesehen ist, der in bekannter Weise über die zu behandelnde Oberfläche gestrichen wird. Es ist dann bevorzugt eine Vorrichtung vorgesehen, mittels derer der Stick oder Stift verlagert werden kann, um diesen nachzuführen, wenn er sich verbraucht und dadurch kleiner wird.

Bevorzugt umfasst der mindestens eine Zusatzstoff ein Parfum. Dieses wird vorzugsweise nach der Inaktivierung insbesondere geruchsrelevanter Moleküle auf die behandelte Oberfläche aufgebracht. Es verleiht dann der Oberfläche einen angenehmen Geruch und kann außerdem gegebenenfalls noch vorhandene Reste üblen Geruchs überdecken beziehungsweise maskieren. Auch ein eventuell verbleibender "technischer" Geruch, der durch das Plasma hervorgerufen wird, kann so überdeckt beziehungsweise maskiert werden.

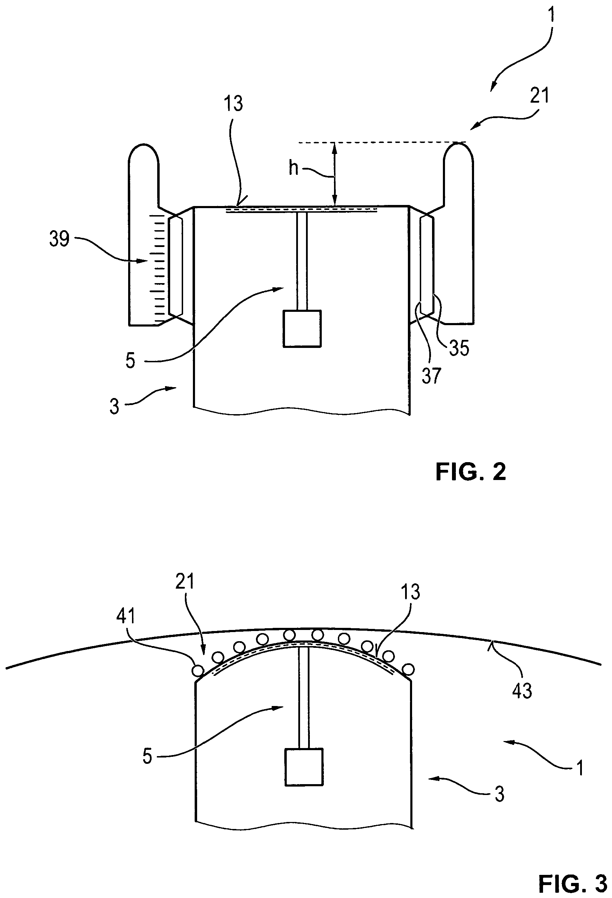

Figur 2 zeigt eine schematische Darstellung eines zweiten Ausführungsbeispiels der Vorrichtung. Gleiche und funktionsgleiche Elemente sind mit gleichen Bezugszeichen versehen, sodass insofern auf die vorangegangene Beschreibung verwiesen wird. Das Abstandshaltemittel 21 ist hier einstellbar ausgebildet. Dazu ist bei dem dargestellten Ausführungsbeispiel an dem Gehäuse 3 ein Außengewinde 35 vorgesehen, welches mit einem Innengewinde 37 des Abstandshaltemittels 21 kämmt. Durch eine Drehung des Abstandshaltemittels 21 relativ zu dem Gehäuse 3 kann so die Höhe h eingestellt werden. Damit variiert auch der Abstand zwischen der Oberfläche 13 und der nicht dargestellten zu behandelnden Oberfläche.

Bei der Behandlung von Oberflächen in Hinblick auf die Vermeidung übler Gerüche oder die Entfernung störender Moleküle sind - wie bereits ausgeführt - insbesondere zwei Mechanismen relevant: Zum einen können insbesondere geruchsrelevante Moleküle durch heiße Elektronen eines Plasmas inaktiviert werden, zum anderen ist es möglich, insbesondere mithilfe reaktiver Spezies des Plasmas im weiteren Sinne auf der Oberfläche vorhandene Bakterien als Quelle störender Moleküle zu inaktivieren beziehungsweise abzutöten. Der erste Mechanismus, welcher im Wesentlichen auf Elektronendissoziation von Molekülen beruht, ist besonders bei kleinen Abständen relevant. Der zweite Mechanismus ist bei etwas größeren Abständen relevant, in denen die vorzugsweise angeregten, jedenfalls aber reaktiven Spezies vorliegen. Mithilfe des einstellbaren Abstandshaltemittels 21 kann zwischen den Wirkmechanismen gewählt werden, beziehungsweise es wird bevorzugt ein Kompromiss zwischen der Wirksamkeit beider Mechanismen eingestellt. In den Extremfällen kann das Abstandshaltemittel 21 so eingestellt werden, dass lediglich der Elektronendissoziationsmechanismus wirksam ist, oder es kann auf eine so große Höhe h eingestellt werden, dass nur noch der bakterizide Effekt wirksam ist. Zwischen diesen Extremen wird bevorzugt eine mittlere Einstellung gewählt.

Um einem Benutzer die Einstellung des Abstandshaltemittels 21 zu erleichtern, ist bevorzugt an diesem eine Skala 39 vorgesehen. Diese kann vorzugsweise Teilstriche umfassen, welche die Höhe h anzeigen. Alternativ können auch Symbole vorgesehen sein, die den jeweils eingestellten Wirkmechanismus beziehungsweise Mischformen hiervon codieren.

Anstelle der Gewinde 35, 37 sind bei einem anderen Ausführungsbeispiel Mittel vorgesehen, mit deren Hilfe das Abstandshaltemittel 21 auf das Gehäuse 3 aufschiebbar und vorzugsweise in festgelegten Positionen arretierbar ist. Beispielsweise kann eine Art Klickraster vorgesehen sein. Solche Verstellvorrichtungen sind zum Beispiel von Haarschneidemaschinen bekannt. Es kann auch eine kontinuierliche Verstellbarkeit des Abstandshaltemittels 21 so vorgesehen sein, dass eine relativ große Reibung zwischen dem Gehäuse 3 und dem Abstandshaltemittel 21 gegeben ist, sodass der Benutzer durch eine Relativverschiebung des Abstandshaltemittels 21 und des Gehäuses 3 eine definierte Höhe h einstellen kann.

Figur 3 zeigt eine schematische Darstellung eines dritten Ausführungsbeispiels der Vorrichtung. Gleiche und funktionsgleiche Elemente sind mit gleichen Bezugszeichen versehen, sodass insoweit auf die vorangegangene Beschreibung verwiesen wird. Bei dem dargestellten Ausführungsbeispiel ist die Oberfläche 13 der Plasmaquelle 5 gekrümmt, nämlich konvex ausgebildet. Bei anderen Ausführungsbeispielen ist eine andere, auch unregelmäßige Krümmung möglich. Eine konvexe Krümmung der Oberfläche 13 hat den Vorteil, dass stets verschiedene Abstände der Oberfläche 13 zu einer zu behandelnden Oberfläche 43 bestehen, falls diese einen von der Oberfläche 13 verschiedenen Krümmungsradius aufweist. So können in verschiedenen Bereichen der Oberfläche 13 beziehungsweise der zu behandelnden Oberfläche 43 verschiedene Wirkmechanismen aktiv sein. Ist die in Figur 3 dargestellte Vorrichtung 1 zylindersymmetrisch ausgebildet, nimmt der Abstand zwischen der Oberfläche 13 und der Oberfläche 43 radial nach innen ab. In einem radial innen liegenden Bereich ist dann zumindest bereichsweise bevorzugt ein Abstand vorhanden, in dem ein ausreichender Fluss heißer Elektronen auf die Oberfläche 43 gewährleistet ist, um dort Moleküle zu inaktivieren. Radial außen ergeben sich vorzugsweise Bereiche, in welchen die bakterizide Wirkung reaktiver Spezies dominiert. Wird die gesamte zu behandelnde Oberfläche 43 mithilfe der Vorrichtung 1 überstrichen, sind beide Wirkmechanismen an allen Stellen der Oberfläche 43 wirksam, ohne dass es einer Veränderung der Vorrichtung 1, beispielsweise einer Einstellung eines Abstandshaltemittels bedarf.

Um einen Mindestabstand zwischen der Oberfläche 13 und der zu behandelnden Oberfläche 43 zu gewährleisten, ist allerdings bevorzugt auch bei diesem Ausführungsbeispiel ein Abstandshaltemittel 21 vorgesehen. Bei dem dargestellten Ausführungsbeispiel umfasst dieses Vorsprünge, die auf der Oberfläche 13 angeordnet sind und von denen hier ein Vorsprung beispielhaft mit dem Bezugszeichen 41 bezeichnet ist. Die Höhe der Vorsprünge 41 über der Oberfläche 13 ist bevorzugt so gewählt, dass eine effiziente Behandlung der zu behandelnden Oberfläche im Rahmen der Elektronendissoziation insbesondere geruchsrelevanter Moleküle gewährleistet ist, wenn die zu behandelnde Oberfläche 43 zumindest bereichsweise auf den Vorsprüngen 41 aufliegt. Bei anderen Ausführungsbeispielen sind andere Abstandshaltemittel 21 vorgesehen: So können beispielsweise Rippen, Gitter, kreisförmige Strukturen oder ähnliche Abstandshaltemittel vorgesehen sein. Es ist auch möglich, die Oberfläche 13 in den Bereichen, in denen Plasma ausgebildet wird, vertieft auszubilden, sodass zwischen den Vertiefungen verbleibende Bereiche der Oberfläche 13 als Abstandshaltemittel 21 wirken.

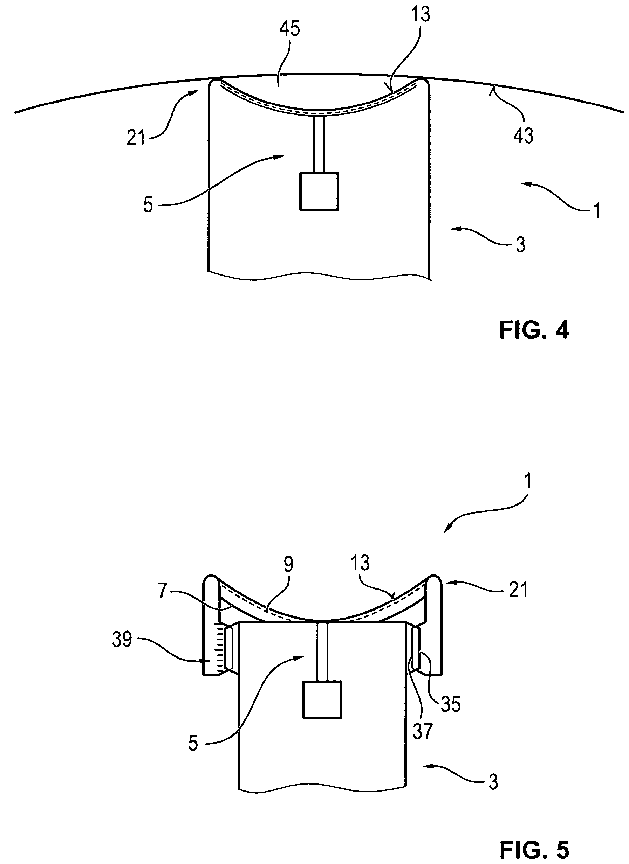

Figur 4 zeigt eine schematische Darstellung eines vierten Ausführungsbeispiels der Vorrichtung 1. Gleiche und funktionsgleiche Elemente sind mit gleichen Bezugszeichen versehen, sodass insofern auf die vorangegangene Beschreibung verwiesen wird. Bei dem dargestellten Ausführungsbeispiel ist die Oberfläche 13 konkav ausgebildet. Dies hat zum einen den Vorteil, dass mithilfe der Vorrichtung 1 entsprechende konkave Oberflächen auf einfache Weise behandelbar sind. Zum anderen ergibt sich aber in Zusammenhang mit einer zu behandelnden Oberfläche 43 der Vorteil, dass der höchste Punkt der Oberfläche 13 quasi selbst ein Abstandshaltemittel 21 realisiert. Die zu behandelnde Oberfläche 43 liegt quasi auf dem höchsten Punkt der Oberfläche 13 auf, sodass der Abstand zum tiefsten Punkt der Oberfläche 13 vorgegeben ist. Dabei kann die Krümmung derselben vorzugsweise so gewählt werden, dass ein bestimmter Wirkmechanismus oder eine bestimmte Verteilung von Wirkmechanismen über der Oberfläche 13 realisiert wird.

Beispielsweise ist es möglich, den tiefsten Punkt der Oberfläche 13 so zu wählen, dass ausschließlich eine Inaktivierung insbesondere geruchsrelevanter Moleküle durch heiße Elektronen stattfindet. Es ist aber auch möglich, den tiefsten Punkt so zu wählen, dass in diesem Bereich bevorzugt oder ausschließlich ein bakterizider Effekt aufgrund reaktiver Spezies auf der zu behandelnden Oberfläche 43 wirksam ist, während sich - bei angenommener Zylindersymmetrie der Vorrichtung 1 - im radial außerhalb gelegenen Bereichen, jedenfalls aber in näher an der Oberfläche 43 angeordneten Bereichen der Oberfläche 13 ein Wirkmechanismus einstellt, bei dem heiße Elektronen zur Inaktivierung insbesondere geruchsrelevanter Moleküle beitragen. Auch bei diesem Ausführungsbeispiel kann also durch Überstreichen der gesamten zu behandelnden Oberfläche 43 mit der Vorrichtung 1 gewährleistet werden, dass beide Wirkmechanismen an jedem Ort der Oberfläche 43 wirksam werden.

Es zeigt sich außerdem, dass bei dem in Figur 4 dargestellten Ausführungsbeispiel die Oberfläche 13 und das Abstandshaltemittel 21, nämlich deren höchster Punkt beziehungsweise deren höchste Umfangslinie - so angeordnet sind, dass durch sie und durch die zu behandelnde Oberfläche 43 ein geschlossenes Volumen 45 einschließbar ist. Wie bereits ausgeführt, hat dies den Vorteil, dass eine besonders rasche Inaktivierung insbesondere geruchsrelevanter Moleküle beziehungsweise auch eine besonders rasche Abtötung beziehungsweise Inaktivierung von Bakterien, die üble Gerüche und/oder andere störende Moleküle bilden, möglich ist, weil keine Störeinflüsse von außen in das geschlossene Volumen 45 dringen können.

Figur 5 zeigt eine schematische Darstellung eines fünften Ausführungsbeispiels der Vorrichtung 1. Gleiche und funktionsgleiche Elemente sind mit gleichen Bezugszeichen versehen, sodass insoweit auf die vorangegangene Beschreibung verwiesen wird. Bei dem dargestellten Ausführungsbeispiel ist die Oberfläche 13 flexibel und vorzugsweise auch elastisch ausgebildet. Beispielsweise kann für diesen Bereich der Plasmaquelle 5 ein Gummi oder gummiartiges elastisches Material ausgewählt werden. Besonders bevorzugt sind die Elektroden 7, 9 in das flexible und/oder elastische Material integriert und vorzugsweise selbst auch flexibel und/oder elastisch ausgebildet.

Bei dem dargestellten Ausführungsbeispiel wirkt die Oberfläche 13 mit dem hier einstellbar ausgebildeten Abstandshalter 21 so zusammen, dass das mit einer nicht dargestellten zu behandelnden Oberfläche einschließbare Volumen einstellbar ist. Wird der Abstandshalter 21 in Figur 5 nach oben verlagert, verlagern sich auch zumindest Wandbereiche der Oberfläche 13 zusammen mit diesem nach oben. Dazu ist die Oberfläche 13 zumindest an einem äußeren Ringbereich mit dem Abstandshalter 21 verbunden. Bevorzugt ist ein zentraler Bereich der Oberfläche 13 mit dem Gehäuse 3 derart verbunden, dass dieser Bereich stets in einer konstanten Position relativ zum Gehäuse 3 angeordnet ist, auch wenn das Abstandshaltemittel 21 verstellt wird. Es verlagern sich dann nur Wandbereiche der Oberfläche 13 gemeinsam mit dem Abstandshaltemittel 21. Insbesondere werden die Wandbereiche bei einer Verlagerung des Abstandshaltemittels 21 nach oben gedehnt und ziehen sich bei dessen Verlagerung nach unten wieder zusammen. Damit zeigt sich insgesamt, dass nicht nur das von der Oberfläche 13 und der zu behandelnden Oberfläche eingeschlossene Volumen einstellbar ist, sondern dass auch der relevante Abstand der zu behandelnden Oberfläche zum tiefsten Punkt der Oberfläche 13 einstellbar ist. Es ist also möglich, zwischen verschiedenen Wirkmechanismen oder Verteilungen von Wirkmechanismen über der Oberfläche 13 in bereits beschriebener Weise auszuwählen.

Zu diesem Zweck ist es auch bei dem hier dargestellten Ausführungsbeispiel möglich, die Skala 39 an dem Abstandshaltemittel 21 vorzusehen.

Die Vorrichtung 1 gemäß Anspruch 5 und insbesondere das Verfahren gemäß Anspruch 1 sind nicht auf die hier dargestellten Ausführungsbeispiele beschränkt, sondern durch die angehängten Ansprüche. Das Verfahren und die Vorrichtung können überall dort eingesetzt werden, wo üble Gerüche, die Anwesenheit störender Moleküle, oder deren Entstehung verhindert werden soll.

Ganz besonders bevorzugt ist die Vorrichtung 1 als Deodorantvorrichtung ausgebildet. Es ist jedenfalls dann auch bevorzugt ein Applikationsmittel 25 vorgesehen, durch welches Zusatzstoffe appliziert werden können. Hierbei handelt es sich bevorzugt um mindestens ein Deodorant und/oder mindestens ein Antitranspirant.

In nicht zur Erfindung gehörender Weise muss das Verfahren allerdings nicht auf die Behandlung von Oberflächen beschränkt sein. Es kann beispielsweise auch in Abzugshauben, insbesondere Dunstabzugshauben, Gasreinigungseinrichtungen, Staubsauger, Föhne, Dampfstrahler, Vorrichtungen zur Durchleitung von Gasen, Dämpfen, Stäuben, Flüssigkeiten oder anderer Fluide, insbesondere Zapfanlagen oder Wasserhähne, Handtrockner oder ähnlichen Vorrichtungen Anwendung finden. Es muss nur sichergestellt sein, dass eine hinreichende Dichte zu inaktivierender Moleküle mit einem hinreichenden Fluss heißer Elektronen beaufschlagt wird, sodass eine nennenswerte Inaktivierung der Moleküle stattfindet. Beispielsweise können enge Kanäle innerhalb einer Durchleitung für Gase, Dämpfe, Stäube, Flüssigkeiten oder andere Fluide vorgesehen sein, innerhalb derer eine entsprechende Inaktivierung insbesondere geruchsrelevanter Moleküle stattfindet.

In diesem Zusammenhang wird eine nicht zur Erfindung gehörende Vorrichtung beschrieben, mit deren Hilfe das Verfahren durchführbar ist, und die einen Fluidstrom durchleitet und/oder erzeugt. Sie umfasst eine Plasmaquelle, welche so ausgebildet und/oder angeordnet ist, dass sie Moleküle, insbesondere geruchsrelevante oder anderweitig störende Moleküle, in dem Fluidstrom inaktivieren kann. Bei dem Fluidstrom handelt es sich bevorzugt um Gase, Dämpfe, Stäube, Flüssigkeiten oder andere Fluide. Die Vorrichtung kann als Abzugshaube, insbesondere Dunstabzugshaube, Gasreinigungseinrichtung, Staubsauger, Föhn, Dampfstrahler, Vorrichtung zur Durchleitung von Flüssigkeiten, insbesondere Zapfanlage, oder Handtrockner ausgebildet sein. Es ist auch möglich, dass die Vorrichtung als Wasserhahn ausgebildet ist. Jedenfalls ist es mit Hilfe der Vorrichtung möglich, geruchsrelevante oder anderweitig störende, gegebenenfalls gesundheitsschädliche Moleküle in dem Fluidstrom zu inaktivieren, bevor dieser seiner eigentlichen Bestimmung zugeführt wird beziehungsweise seinen Bestimmungsort erreicht. Besonders bevorzugt ist die Elektrode der Plasmaquelle, in deren Bereich das Plasma erzeugt wird, auf einer Oberfläche angeordnet oder in diese eingebettet, welche unmittelbaren Kontakt zu dem Fluidstrom hat. Um sicherzustellen, dass eine hinreichende Dichte zu inaktivierende Moleküle mit einem hinreichenden Fluss heißer Elektronen beaufschlagt wird, sind bevorzugt enge Kanäle zur Durchleitung des Fluidstroms vorgesehen. In diesem Fall ist die entsprechende Elektrode bevorzugt an einer Oberfläche dieser Kanäle angeordnet oder in diese eingebettet. Das Plasma wird so unmittelbar auf einer Kanaloberfläche gebildet, so dass störende Moleküle effizient inaktiviert werden können. Das durch die Plasmaquelle erzeugte Plasma entfaltet innerhalb der Vorrichtung selbstverständlich auch eine bakterizide beziehungsweise desinfizierende und/oder sterilisierende Wirkung. Auch Keime, Pilze, Sporen, Bakterien und Viren werden daher effizient in dem Fluidstrom inaktiviert.

Es ist bei allen Ausführungsformen und Ausführungsbeispielen möglich, dem Plasma selbst Additive beizufügen, die in Zusammenhang mit dem Plasma eine besondere Wirkung entfalten. Dies können beispielsweise Gase, Katalysatoren und/oder medizinisch aktive Wirkstoffe sein, die entweder zu einer Aktivierung von Komponenten des Plasmas beitragen, durch das Plasma selbst aktiviert werden oder bestimmte Reaktionen katalysieren beziehungsweise auf der zu behandelnden Oberfläche bestimmte Wirkungen herbeiführen. Diese Additive können vor, hinter oder zwischen den Elektroden in einen Diffusions- oder Strömungspfad des Plasmas eingebracht werden. Es ist auch möglich, diese Additive mithilfe des Applikationsmittels 25 auf die zu behandelnde Oberfläche aufzubringen.

Erfindungsgemäß umfasst eine zu behandelnde Oberfläche textiles Material, Keramik, Kunststoff und/oder Leder. So kann beispielsweise Kleidung von üblen Gerüchen und/oder störenden Molekülen mithilfe des Verfahrens und/oder der Vorrichtung 1 befreit werden. Insbesondere in Zusammenhang mit textilem Material, beispielsweise Kleidung, und generell in Zusammenhang mit Materialien, welche Fasern umfassen, ist der Begriff "Oberfläche" hier in einem weiteren Sinne zu verstehen. Das Plasma und die heißen Elektronen weisen bei solchen Materialien eine gewisse Eindringtiefe auf, so dass nicht ausschließlich die unmittelbare Oberfläche behandelt wird, sondern eine Tiefenwirkung besteht, die durchaus die gesamte behandelte Lage des Materials umfassen kann. So können solche Materialien mithilfe der Vorrichtung 1 und des Verfahrens effektiv von üblen Gerüchen und/oder störenden Molekülen befreit werden.

Die Vorrichtung 1 und das Verfahren sind auch in Zusammenhang mit WC-Schüsseln, Waschbecken oder anderen keramischen Vorrichtungen verwendbar, an deren Oberflächen üble Gerüche oder störende Moleküle entstehen können. Dies gilt insbesondere auch für Kunststoff, beispielsweise im Küchenbereich, oder auch Leder, beispielsweise im Wohnbereich. Auch Teppiche und Fußböden, Treppenstufen, Wände, allgemein alle Haushaltsoberflächen, aber auch gewerbliche Oberflächen sind mithilfe des Verfahrens beziehungsweise der Vorrichtung 1 behandelbar. Ein vorzugsweise mithilfe des Applikationsmittels 25 auftragbarer Zusatzstoff kann dann bevorzugst ein Reinigungs- oder Pflegemittel sein.

Schließlich sind die Vorrichtung 1 und das Verfahren insbesondere auch in Zusammenhang mit Schuhen anwendbar. Bevorzugt werden die Vorrichtung 1 und das Verfahren im Inneren von Schuhen angewendet. Dazu kann die Vorrichtung 1 vorzugsweise die Form eines Schuhspanners oder einer Einlegesohle aufweisen, als Schuhspanner oder Einlegesohle ausgebildet sein, oder auch in einen Schuhspanner oder eine Einlegesohle integriert sein. Beispielsweise ist es möglich, in der Oberfläche eines Schuhspanners Vertiefungen vorzusehen, in denen das Plasma erzeugt wird. Die unvertieften Oberflächenbereiche fungieren dann als Abstandshaltemittel 21. Ebenso ist es möglich, Vorsprünge als Abstandshaltemittel 21 an der Oberfläche vorzusehen. Bevorzugt folgt die Oberfläche der Vorrichtung 1, an der Plasma erzeugt wird, der Kontur des Schuhs, so dass eine großflächige Behandlung gegeben ist. Besonders bevorzugt wird allerdings eine dem Fuß zugewandte Seite der Schuhsohle behandelt, so dass es bei einem Ausführungsbeispiel genügen kann, nur in diesem Bereich eine Plasmaquelle vorzusehen. Insbesondere kann im unteren Bereich eines Schuhspanners in dem Zwischenraum zwischen einer der Sohle zugewandten Fläche des Schuhspanners und der Sohle Plasma zur Inaktivierung übler Gerüche oder anderer störender Moleküle erzeugt werden.

Die bakterizide Wirkung der Vorrichtungen und des Verfahrens wurde insbesondere in Zusammenhang mit dem für üblen Körpergeruch verantwortlichen Bakterium Corynebacterium jeikeium getestet und nachgewiesen. Bezüglich der Bekämpfung übler Gerüche wurde ein so genannter Schnüffeltest durchgeführt, in welchem Probanden mit einer übel riechenden Lösung behandelte Hautbereiche und Textilien im Rahmen eines Blindversuchs als Riechprobe dargereicht wurden, wobei die Proben teilweise mit der Vorrichtung beziehungsweise mit dem Verfahren behandelt und teilweise unbehandelt waren. Es ergab sich dabei eindeutig, dass die behandelten Hautbereiche beziehungsweise Textilien besser rochen als die unbehandelten. Ein eventuell wahrnehmbarer "technischer" Geruch, der durch das Plasma erzeugt wird, kann durch mindestens einen geeigneten Zusatzstoff maskiert werden.

Damit zeigt sich insgesamt, dass das Verfahren und die Vorrichtungen insbesondere geeignet sind, üble Gerüche zu reduzieren, deren Entstehung zu verhindern beziehungsweise zu verlangsamen und letztlich auch die Wachstumsbedingungen für geruchsbildende Bakterien vorzugsweise durch Inaktivierung von deren Nahrung zu reduzieren. In Kombination mit Zusatzstoffen ist ein zusätzlicher Maskierungseffekt übler Gerüche möglich. Damit schließen das Verfahren und die Vorrichtungen eine Lücke, weil es bisher nur möglich war, die Ursache für die Entstehung übler Gerüche zu behandeln, nicht jedoch, die üblen Gerüche selbst zu beseitigen. Ebenso ist eine Inaktivierung anderer störender Moleküle möglich.

Insbesondere ist alles, was bezüglich des Verfahrens und der Vorrichtungen in Zusammenhang mit geruchsrelevanten Molekülen ausgeführt wurde, entsprechend auch in Zusammenhang mit der Inaktivierung anderer störender Moleküle relevant.

## Patentansprüche

1. Verfahren zur Inaktivierung von Molekülen auf einer Oberfläche (43), mit folgenden Schritten:
- Erzeugen eines Plasmas mittels einer Plasmaquelle (5), die zwei Elektroden (7, 9) aufweist, wobei eine erste Elektrode (7) der Elektroden (7, 9) mit einer Wechselspannung beaufschlagt wird und eine zweite Elektrode (9) der Elektroden (7, 9) geerdet ist, wobei die Elektroden (7, 9) durch ein Dielektrikum voneinander beabstandet sind, und wobei eine der Elektroden (7, 9) an einer der zu behandelnden Oberfläche (43) zugewandten Oberfläche (13) vorgesehen ist, und wobei zwischen den Elektroden (7, 9) eine Entladung gezündet wird, sodass auf der Oberfläche (13) ein Plasma gebildet wird,
wobei das Plasma auf der Oberfläche (13) in einem Abstand von 0 bis 1 mm zu der zu behandelnden Oberfläche (43) erzeugt wird, wobei das Plasma heiße Elektronen, die eine mittlere kinetische Energie von mindestens einem Elektronenvolt aufweisen, umfasst, wobei die zu behandelnde Oberfläche (43) ausgewählt ist aus textilem Material, Keramik, Kunststoff oder Leder.

2. Verfahren nach Anspruch 1, wobei zusätzlich Bakterien, vorzugsweise geruchsbildende Bakterien, durch das Plasma abgetötet werden.

3. Verfahren nach einem der vorhergehenden Ansprüche, wobei ein Zusatzstoff auf die zu behandelnde Oberfläche (43) aufgebracht wird, und/oder der Zusatzstoff ein Deodorant und/oder Antitranspirant ist.

4. Verfahren nach einem der vorhergehenden Ansprüche, wobei Allergene, Proteinmoleküle oder Prionen inaktiviert werden.

5. Vorrichtung zur Durchführung eines Verfahrens zur Inaktivierung von Molekülen auf einer Oberfläche (43) nach einem der Ansprüche 1 bis 4 mit
- einem Gehäuse (3);
- einer dem Gehäuse (3) zugeordneten Plasmaquelle (5), und
- mindestens einem Abstandshaltemittel (21),
wobei die Plasmaquelle (5) eine Spannungsquelle (11) und zwei mit der Spannungsquelle (11) verbundene Elektroden (7, 9) aufweist, wobei die Spannungsquelle (11) eingerichtet ist, um eine erste Elektrode (7) der Elektroden (7, 9) mit einer Wechselspannung zu beaufschlagen und eine zweite Elektrode (9) der Elektroden (7, 9) zu erden, wobei die Elektroden (7, 9) durch ein Dielektrikum voneinander beabstandet sind, und wobei eine der Elektroden (7, 9) an einer der zu behandelnden Oberfläche (43) zugewandten Oberfläche (13) vorgesehen ist, wobei zwischen den Elektroden (7, 9) durch Beaufschlagen der ersten Elektrode (7) mit der Wechselspannung eine Entladung gezündet werden kann, sodass auf der Oberfläche (13) ein Plasma gebildet wird, und dass das mindestens eine Abstandshaltemittel (21) so an dem Gehäuse (3) und/oder der Plasmaquelle (5) vorgesehen ist, dass zumindest bereichsweise ein Abstand von 0 bis 1 mm zwischen der Oberfläche (13) und der zu behandelnden Oberfläche (43) einhaltbar ist.

6. Vorrichtung nach Anspruch 5, wobei das Abstandshaltemittel (21) einstellbar ausgebildet ist.

7. Vorrichtung nach einem der Ansprüche 5 oder 6, wobei die Oberfläche (13) der Plasmaquelle (5) vorzugsweise gekrümmt, vorzugsweise konvex oder konkav, ausgebildet ist.

8. Vorrichtung nach einem der Ansprüche 5 bis 7, wobei die Oberfläche (13) der Plasmaquelle (5) und das Abstandshaltemittel (21) so angeordnet sind, dass durch sie und die zu behandelnde Oberfläche (43) ein geschlossenes Volumen (45) einschließbar ist.

9. Vorrichtung nach einem der Ansprüche 5 bis 8, wobei die Oberfläche (13) der Plasmaquelle (5) flexibel und/oder elastisch ausgebildet ist.

10. Vorrichtung nach einem der Ansprüche 5 bis 9, wobei die flexible und/oder elastische Oberfläche (13) der Plasmaquelle (5) mit dem einstellbaren Abstandshalter (21) so zusammenwirkt, dass das mit der zu behandelnden Oberfläche (43) einschließbare Volumen (45) einstellbar ist.

11. Vorrichtung nach einem der Ansprüche 5 bis 10, wobei die Vorrichtung ein Applikationsmittel (25) zum Aufbringen eines vorzugsweise flüssigen Zusatzstoffs auf die zu behandelnde Oberfläche (43) aufweist, wobei das Applikationsmittel (25) bevorzugt ein Spray, eine Rollervorrichtung oder eine Stickvorrichtung umfasst.

12. Vorrichtung nach einem der Ansprüche 5 bis 11, wobei die Vorrichtung die Form eines Schuhspanners oder einer Einlegesohle aufweist, als Schuhspanner oder Einlegesohle ausgebildet oder in einen Schuhspanner oder eine Einlegesohle integriert ist.

## Claims

1. Method for inactivating molecules on a surface (43), comprising the following steps:
- Generating a plasma by means of a plasma source (5) comprising two electrodes (7, 9), wherein an alternating voltage is applied to a first electrode (7) of the electrodes (7, 9), and a second electrode (9) of the electrodes (7, 9) is grounded, wherein the electrodes (7, 9) are spaced apart from one another by a dielectric, and wherein one of the electrodes (7, 9) is provided on a surface (13) facing the surface (43) to be treated, and wherein a discharge is ignited between the electrodes (7, 9) so that a plasma is formed on the surface (13),
wherein the plasma is generated on the surface (13) at a distance of 0 to 1 mm from the surface (43) to be treated, wherein the plasma comprises hot electrons having an average kinetic energy of at least one electron volt, wherein the surface (43) to be treated is selected from textile material, ceramic, plastic or leather.

2. Method according to claim 1, wherein additionally bacteria, preferably odor-forming bacteria, are killed by the plasma.

3. Method according to one of the preceding claims, wherein an additive is applied to the surface (43) to be treated, and/or the additive is a deodorant and/or an antitranspirant.

4. Method according to one of the preceding claims, wherein allergens, protein molecules or prions are inactivated.

5. Device for carrying out a method for inactivating molecules on a surface (43) according to one of the claims 1 to 4, comprising
- a housing (3);
- a plasma source (5) associated with the housing (3), and
- at least one spacer means (21),
wherein the plasma source (5) comprises a voltage source (11) and two electrodes (7, 9) connected to the voltage source (11), wherein the voltage source (11) is adapted to apply an alternating voltage to a first electrode (7) of the electrodes (7, 9) and to ground a second electrode (9) of the electrodes (7, 9), wherein the electrodes (7, 9) are spaced apart from one another by a dielectric, and wherein one of the electrodes (7, 9) is provided on a surface (13) facing the surface (43) to be treated, wherein a discharge can be ignited between the electrodes (7, 9) by applying the alternating voltage to the first electrode (7), so that a plasma is formed on the surface (13), and in that the at least one spacer means (21) is provided on the housing (3) and/or the plasma source (5) in such a way that a distance of 0 to 1 mm can be maintained at least in regions between the surface (13) and the surface (43) to be treated.

6. Device according to claim 5, wherein the spacer means (21) is configured to be adjustable.

7. Device according to one of the claims 5 or 6, wherein the surface (13) of the plasma source (5) is formed preferably curved, preferably convex or concave.

8. Device according to one of the claims 5 to 7, wherein the surface (13) of the plasma source (5) and the spacer means (21) are arranged such that a closed volume (45) can be enclosed by them and the surface (43) to be treated.

9. Device according to one of the claims 5 to 8, wherein the surface (13) of the plasma source (5) is configured to be flexible and/or elastic.

10. Device according to one of the claims 5 to 9, wherein the flexible and/or elastic surface (13) of the plasma source (5) cooperates with the adjustable spacer (21) such that the volume (45) enclosable with the surface (43) to be treated is adjustable.

11. Device according to one of the claims 5 to 10, wherein the device comprises an application means (25) for applying a preferably liquid additive to the surface (43) to be treated, the application means (25) preferably comprising a spray, a roller device or a stick device.

12. Device according to one of the claims 5 to 11, wherein the device comprises the form of a shoe tree or an inlay sole, is configured as a shoe tree or an inlay sole or is integrated into a shoe tree or an inlay sole.

## Revendications

1. Procédé visant à désactiver des molécules sur une surface (43), comportant les étapes :
- générer un plasma au moyen d'une source de plasma (5) comportant deux électrodes (7, 9), une tension alternative étant appliquée à une première électrode (7) des électrodes (7, 9) et une deuxième électrode (9) des deux électrodes (7, 9) étant reliée à la terre, les électrodes (7, 9) étant espacées l'une de l'autre par un diélectrique, et une des électrodes (7, 9) étant prévue sur une surface (13) tournée vers la surface (43) à traiter, et une décharge étant déclenchée entre les électrodes (7, 9) pour ainsi former un plasma sur la surface (13), dans lequel
le plasma est généré sur la surface (13) de manière à ce qu'il se trouve à une distance de 0 à 1 mm de la surface (43) à traiter, dans lequel le plasma comprend des électrons chauds dont l'énergie cinétique moyenne est d'au moins un électronvolt, dans lequel la surface (43) à traiter est choisie parmi une matière textile, une céramique, une matière plastique ou du cuir.

2. Procédé selon la revendication 1, dans lequel des bactéries, s'agissant préférentiellement de bactéries provoquant des odeurs, sont éliminées par ledit plasma.

3. Procédé selon l'une des revendications précédentes, dans lequel un additif est appliqué sur la surface (43) à traiter, et/ou ledit additif est un agent désodorisant et/ou anti-transpirant.

4. Procédé selon l'une des revendications précédentes, dans lequel des allergènes, des molécules de protéine ou des prions sont désactivés.

5. Dispositif destiné à mettre en oeuvre un procédé visant à désactiver des molécules sur une surface (43) selon l'une des revendications 1 à 4, comportant
- un boîtier (3);
- une source de plasma (5) associée au boîtier (3), et
- au moins un espaceur (21); dans lequel
la source de plasma (5) comporte une source de tension (11) et deux électrodes (7, 9) reliées à la source de tension (11), dans lequel la source de tension (11) est conçue pour appliquer une tension alternative à une première électrode (7) des électrodes (7, 9) et à relier à la terre une deuxième électrode (9) des électrodes (7, 9), dans lequel les électrodes (7, 9) sont espacées l'une de l'autre par un diélectrique, et dans lequel une des électrodes (7, 9) est disposée sur une surface (13) tournée vers la surface (43) à traiter, dans lequel une décharge peut être déclenchée entre les électrodes (7, 9) en appliquant ladite tension alternative à la première électrode (7) pour ainsi former un plasma sur la surface (13), et dans lequel l'au moins un espaceur (21) est disposé sur le boîtier (3) et/ou sur la source de plasma (5) de manière à ce que, sur certaine parties, une distance de 0 à 1 mm puisse être maintenue entre la surface (13) et la surface (43) à traiter.

6. Dispositif selon la revendication 5, dans lequel l'espaceur (21) est réalisé de manière à être réglable.

7. Dispositif selon l'une des revendications 5 ou 6, dans lequel la surface (13) de la source de plasma (5) est réalisée préférentiellement de manière à ce qu'elle soit courbée, de préférence convexe ou concave.

8. Dispositif selon l'une des revendications 5 à 7, dans lequel la surface (13) de la source de plasma (5) et l'espaceur (21) sont disposés de manière à ce que, conjointement avec la surface (43) à traiter, ils puissent ensemble confiner un volume clos (45).

9. Dispositif selon l'une des revendications 5 à 8, dans lequel la surface (13) de la source de plasma (5) est réalisée de manière à ce qu'elle soit flexible et/ou élastique.

10. Dispositif selon l'une des revendications 5 à 9, dans lequel la surface (13) flexible et/ou élastique de la source de plasma (5) coopère avec l'espaceur (21) réglable de manière à ce que l'on puisse régler le volume (45) lequel pourra être confiné par la surface (43) à traiter.

11. Dispositif selon l'une des revendications 5 à 10, dans lequel ledit dispositif comporte un moyen d'application (25) permettant d'appliquer un additif, préférentiellement sous une forme liquide, sur la surface (43) à traiter, dans lequel le moyen d'application (25) comprend préférentiellement un spray, un dispositif à bille ou un dispositif de stick.

12. Dispositif selon l'une des revendications 5 à 11, dans lequel ledit dispositif a la forme d'un embauchoir ou d'une semelle de propreté, est réalisé en tant qu'embauchoir ou semelle de propreté ou est intégré à un embauchoir ou à une semelle de propreté.
